# EUROPÄISCHE PATENTSCHRIFT

(11) **EP 2 228 175 B1**
(45) Veröffentlichungstag und Bekanntmachung des Hinweises auf die Patenterteilung: **19.06.2013**
(21) Anmeldenummer: 09155004.6
(22) Anmeldetag: 12.03.2009
(51) Int. Cl.: B24C 11/00, A61C 3/025, A61Q 11/00, B24C 7/00

(54) **Verwendung eines Pulvers oder Pulvergemisches zur Herstellung eines Mittels zur Pulverstrahlreinigung von Zahnoberflächen**
Use of a powder or powder mixture for the production of a medium for pulverulent jet cleaning of dental surfaces
Utilisation d'un poudre ou d'un mélange de poudre pour la production d'un agent pour le nettoyage par jet pulvérulent de surfaces dentaires

(43) Veröffentlichungstag der Anmeldung: 15.09.2010
(73) Patentinhaber: Ferton Holding SA, 2800 Delemont (CH)
(72) Erfinder: Donnet, Marcel, 01630 Saint Jean de Gonville (FR); Wittmann, Jörg, 64285 Darmstadt (DE)
(74) Vertreter: Müller Schupfner & Partner

(56) Entgegenhaltungen:
- EP-A- 0 630 573
- EP-A- 1 374 871
- EP-A- 1 905 430
- EP-A- 1 972 675
- WO-A-98/40434
- DE-A1- 10 026 718
- DE-A1- 19 804 065
- JP-A- 2000 204 354

## Beschreibung

Die vorliegende Erfindung betrifft die Verwendung eines Pulvers bzw. eines Pulvergemischs zur Herstellung eines Mittels zur Pulverstrahlreinigung von Zahnoberflächen, bevorzugt Dentin. Ein Pulver zum Pulverstrahlen mit einem Pulverstrahlgerät bzw. ein Pulvergemisch zum Pulverstrahlen mit einem Pulverstrahlgerät, ist beispielsweise in der EP 1 159 929 oder in der DE 10 026 718 beschrieben.

Solche Pulver sind bereits aus der US 3 882 638 oder der US 4 174 571 bekannt, wobei die Fortentwicklung der zweitgenannten Druckschrift darauf abzielt, besonders schonende Pulver wie Glutamat oder Natriumgluconat anzugeben, die gegenüber den bis dahin bekannten Pulvern aus Natriumbicarbonat oder Islandspat eine geringere Abrasivität aufweisen und somit empfindliche Oberflächen, wie beispielsweise Zahnwurzeloberflächen, aber auch Zahnschmelzoberflächen, weniger stark beeinträchtigen. Bevorzugte Korngrößen werden mit 140 µm bis 200 µm, bei stärker abrasiv wirkenden Pulvern auch mit 20 µm bis 70 µm angegeben.

Bei der Bearbeitung von empfindlichen Zahnoberflächen, wie Wurzeldentin, oder andere weniger stark mineralisierte Zahnoberflächen mittels eines Pulverstrahlgeräts empfiehlt es sich, Pulver mit einer Dichte von nicht mehr als 2,0 g/cm³ und einer mittleren Korngröße von nicht mehr als 45 µm zu verwenden. Die WO 00/53154 offenbart solche besonders schonenden Pulver zum Reinigen von subgingivalen Zahnoberflächen, wie beispielsweise Wurzeldentin. Gleiches offenbart auch die US 6 126 444, wonach empfindliche, insbesondere subgingivale Zahnwurzeloberflächen mit einem Pulver aus beispielsweise kristalliner Zellulose mit mittleren Korngrößen von 6, 15, 20, 50 oder auch 120 µm bearbeitet wurden und die Reinigungswirkung ähnlich gut ist wie bei der Verwendung eines Pulvers aus Natriumbicarbonat, wobei der Abtrag von Wurzeldentin erheblich geringer ist. Während die US 6 126 444 wasserunlösliche Stoffe offenbart, zeigt die WO 00/53154 wasserlösliche Stoffe wie Aminosäuren, Zucker oder organische Säuren sowie deren Salze, die den Vorteil haben, dass sich das Pulver bei der Anwendung am Zahn im Wasser löst und sich insbesondere in den Zahnfleischtaschen nicht ansammelt, wo wasserunlösliche Stoffe ein unangenehmes Gefühl hervorrufen und ggf. sogar zu einer Entzündung der Zahnfleischtasche bzw. des Zahnfleischs beitragen können. Allerdings haben diese Pulver den Nachteil, dass die Herstellung aufgrund eines oder mehrerer Mahl- und Siebvorgänge teuer und umständlich ist.

Während Pulver, die aus kleineren Partikeln bestehen, einen geringeren Impuls beim Auftreffen auf die Zahnoberfläche haben und somit auch weniger Material abtragen, gilt Gleiches für leichtere Stoffe, die ebenfalls einen geringeren Impuls haben und somit weniger Material abtragen. Aus der US 6 126 444 wird allerdings ersichtlich, dass Pulver mit einer kleineren mittleren Korngröße ähnlich gute Reinigungswirkungen erzielen, wie Pulver mit größeren mittleren Korngrößen, weswegen die Korngröße allein oder die Dichte allein noch keinen Aufschluss darüber gibt, ob das Pulver - bei etwa gleich guter Reinigungswirkung - stärker oder schwächer abrasiv an der Zahnoberfläche einwirkt.

US 5 810 587 offenbart eine weitere Zusammensetzung zur Reinigung von empfindlichen Zahnoberflächen mittels Pulverstrahlgeräten, wobei einerseits eine gute Reinigungswirkung zur Entfernung von Zahnbelägen erzielt, gleichzeitig aber eine möglichst geringe Abrasivität bei der Beaufschlagung von empfindlichen Zahnoberflächen erreicht werden soll. Die US 5 810 587 empfiehlt die Verwendung von sehr feinteiligen Stoffen, die zu größeren "Schneebällen" zusammengesetzt sind und beim Auftreffen auf die Zahnoberfläche zerbersten. Teilchen mit Größen von 0,01 bis 5 µm werden zu größeren Kugeln geformt, die Dimensionen von 10 bis 200 µm aufweisen und beim Auftreffen auf die Zahnoberfläche zerbersten. Als Rohmaterial wird Gibbsit empfohlen.

Im Wesentlichen sind fünf Faktoren für die Abrasivität eines Pulvers für die Pulverstrahlanwendung maßgeblich. Neben der Teilchengeschwindigkeit, Härte und Bruchfestigkeit der Teilchen sind auch die Oberflächenbeschaffenheit und die Masse maßgeblich. Bei gleicher Masse haben große Teilchen mit einer geringeren Dichte in etwa die gleiche Abrasivität, wie kleinere Teilchen mit einer größeren Dichte. Kleinere Teilchen lassen sich allerdings in den bekannten Pulverstrahlgeräten weniger gut verarbeiten. In der Regel befindet sich ein solches Pulver innerhalb einer Pulverkammer, wie dies die EP 1 159 929 zeigt. Luft wird in diese Pulverkammer eingeblasen, um das Pulver aufzuwirbeln, wobei das daraus entstehende Luft-/Pulvergemisch einer Düse zugeführt wird, zu der in der Regel über eine separate Zuleitung auch Wasser zugeführt wird. Am Düsenende tritt das Pulver-/Luftgemisch von einem Wasserstrahl umgeben aus und trifft auf die Zahnoberfläche, die in der Regel zwischen 1 mm und 5 mm vom Düsenaustritt entfernt angeordnet ist.

Aus der US 4 174 571 ist es bekannt, wasserlösliche Pulver zu verwenden, die auch geschmacklich vom Menschen nicht als unangenehm empfunden werden. Auch ein Verklumpen innerhalb der Pulverkammer sollte vermieden werden, weswegen das Pulver auch nicht hygroskopisch sein sollte. Diese Eigenschaft lässt sich auch durch die Zugabe weiterer feinteiliger Substanzen, wie pyrogener Kieselsäure, verbessern.

Weitere Kriterien für die Auswahl geeigneter Strahlmittel sind die Misch- und Flugeigenschaften der verwendeten Teilchen. Bei der Verwirbelung in der Pulverkammer ist darauf zu achten, dass eine konstante Beladung des austretenden Luftstrahls erfolgt, und zwar unabhängig von der eingefüllten Pulvermenge in der Pulverkammer. Dies wird nur dann gewährleistet, wenn das Pulver gleichmäßig vom Luftstrom durchmischt wird und die Rieselfähigkeit beispielsweise durch die Bildung von verdichteten Arealen nicht eingeschränkt ist. Kleinere Teilchen neigen eher zur Bildung solcher verdichteter Areale als größere Teilchen, die aufgrund ihrer meist größeren Masse allerdings oft weniger geeignet sind, durch den Luftstrahl aufgewirbelt zu werden. Die Mischfähigkeit ist darüber hinaus abhängig von dem verwendeten Stoff. Gleichzeitig sollte das Pulver von den bereits sich im Einsatz befindlichen Pulverstrahlgeräten verarbeitet werden können und die Herstellung des Pulvers sollte möglichst kostengünstig erfolgen.

Des Weiteren sollte ein Pulver zum Pulverstrahlen eine möglichst geringe Korrosionsneigung haben und die Strahldüse möglichst wenig verschleißen. Das Pulver sollte in der bevorzugten Größe möglichst direkt und ohne weitere Mahlvorgänge herstellbar und hautfreundlich sein. Das Pulver sollte für den Menschen gut verträglich und wenn möglich, geschmacklich akzeptabel sein. Um aufwendige Zulassungsverfahren abzukürzen oder zu vermeiden, sollte das Pulver möglichst wenig Nebenwirkungen und leicht bekömmlich sein.

Der vorliegenden Erfindung liegt somit die Aufgabe zu Grunde, die vorerwähnten Nachteile herkömmlicher Pulver zu vermeiden, während gleichzeitig die vorerwähnten Anforderungen möglichst umfassend erfüllt werden.

Diese Aufgabe wird durch die Merkmale der unabhängigen Ansprüche 1 und 7 gelöst. Vorteilhafte Ausgestaltungen der vorliegenden Erfindung sind in den Unteransprüchen beansprucht und dort gekennzeichnet.

Die vorliegende Erfindung hat überraschenderweise festgestellt, dass praktisch alle vorgenannten Anforderungen an ein solches Pulver erfüllt werden können, wenn ein Alditol als Pulver verwendet wird. Alditole sind acyclische Polyole mit der Formel HOCH₂[CH(OH)]ₙCH₂OH, die sich strukturell von den Kohlenhydraten ableiten. Sie werden auch als Zuckeralkohole bezeichnet.

Für die vorliegende Erfindung besonders bevorzugte Zuckeralkohole sind Mannit, Erythrit, Xylitol, Sorbit oder Treit. Alditole schmecken süß, sind nicht kariogen und zeigen eine abführende Wirkung erst bei einem Konsum von mehr als 20 bis 30 g/Tag, können also ohne Weiteres für die Pulverstrahlreinigung von Zahnoberflächen eingesetzt werden. Da Alditole als Zuckeraustauschstoffe in diätetischen Lebensmitteln eingesetzt werden, erhöhen sie den Blutzuckerspiegel nicht und benötigen kein Insulin, um abgebaut zu werden. Sie sind daher auch für Diabetiker geeignet und können somit ohne Weiteres für die Pulverstrahlanwendung eingesetzt werden.

Als besonders bevorzugte Alditole haben sich Mannitol und Erythritol herausgestellt.

Das Mannitol ist ein Alditol mit vier CH(OH)-Gruppen und wird als Zuckeraustauschstoff (E 421) sowie als Arzneimittel bei der Tablettenherstellung eingesetzt. Es ist ein farbloses, süß schmeckendes Kristall und besitzt eine Dichte von etwa 1,52 g/cm³. Durch seine leichte Löslichkeit in Wasser eignet es sich hervorragend für die Pulverstrahlreinigung, da es nach dem Auftreffen auf der Zahnoberfläche gelöst und ausgespült werden kann. Die molare Masse von Mannitol beträgt 182,17 g/mol. Die Summenformel lautet C₆H₁₄O₆. Handelsüblich ist Mannitol unter den Marken Osmofundin^{®}, Osmosteril^{®} oder auch als Bronchitol^{®} erhältlich.

Mannitol hat im Gegensatz zu den bisher bekannten Pulverarten, wie Glycin, Natriumgluconat oder Natriumhydrogencarbonat mehrere Vorteile. Mannitol verschleißt im Vergleich zu Glycin die Strahldüse weniger stark, hat aber im Vergleich zu Glycin bei ähnlicher Teilchengröße eine vergleichbare Abrasivität. Auch reinigt Mannitol die Zahnoberfläche besser als Glycin, ohne empfindliches Dentin zu beschädigen. Gleichzeitig hat sich herausgestellt, dass sich Mannitol im Pulverbehälter sehr gut mit Luft vermischen lässt und eine gleichmäßige Verwirbelung stattfindet. Es staubt trotz geringer Teilchengröße weniger leicht als beispielsweise Glycin und lässt sich somit besser vermischen und gleichmäßiger der Düse des Pulverstrahlgeräts zuführen. Dadurch verbessert sich die Gleichmäßigkeit der Zahnbehandlung.

Des Weiteren ist Mannitol als Pulver in den gewünschten Größen direkt herstellbar und muss nicht erst, wie beispielsweise Natriumhydrogencarbonat oder Glycin, auf die gewünschte Größe zermahlen und gesiebt werden. Des Weiteren ist die Verträglichkeit von Mannitol deutlich besser. Glycin ist zytotoksisch und kann bei einem Kontakt mit der Haut zu Irritationen führen. Mannitol ist ein Zuckeraustauschstoff und kann als Lebensmittel eingesetzt werden. Es ist wasserlöslich, klumpt nicht und schmeckt süßlich.

Die bevorzugte mittlere Korngröße des erfindungsgemäßen Pulvers sollte nicht größer sein als 45 µm, bevorzugt nicht größer als 35 µm, besonders bevorzugt etwa zwischen 10 µm und etwa 30 µm liegen. Eine geeignete mittlere Korngröße von Mannitol-Pulver für die Reinigung empfindlicher Zahnoberflächen liegt bei etwa 20 µm bis 30 µm.

Das erfindungsgemäße Pulver hat eine Dichte von nicht mehr als 2,0 g/cm³, bevorzugt nicht mehr als 1,8 g/cm³, besonders bevorzugt nicht mehr als 1,55 g/cm³. Besonders bevorzugte Dichten liegen zwischen 1,4 g/cm³ und 1,5 g/cm³. Des Weiteren hat eine besondere Ausführungsform des erfindungsgemäßen Pulvers eine Mohssche Härte von nicht mehr als 5, bevorzugt von nicht mehr als 3,5, besonders bevorzugt von nicht mehr als 2,5. Des Weiteren hat das erfindungsgemäße Pulver eine molare Masse von nicht weniger als 90 g/mol, bevorzugt nicht weniger als 110 g/mol, besonders bevorzugt nicht weniger als 120 g/mol. Langkettige Kohlenwasserstoffe bilden dabei Teilchen von geringerer Dichte, d. h., mit einer größeren Angriffsfläche bei gleicher Masse, wodurch sich die Verwirbelungseigenschaften in der Wirbelkammer verbessern.

Während sich Glycin bei einer Teilchengröße von weniger als 20 µm in der Pulverkammer handelsüblicher Geräte nicht mehr zuverlässig verwirbeln lässt und selbst bei der Zugabe von Mitteln zur Steigerung der Rieselfähigkeit, wie pyrogener Kieselsäure, verdichtete Areale bildet und somit keine ausreichende Anreicherung des Luftstrahls mit dem Strahlmittel erlaubt, zeigt sich dies bei der Verwendung eines Pulvers aus beispielsweise Erythritol nicht.

Erythritol besitzt zwei CH(OH)-Gruppen und wird ebenfalls als Zuckeraustauschstoff (E 968) eingesetzt. Es ist bei Zimmertemperatur fest, besitzt eine Dichte von 1,45 g/cm³ und ist in Wasser löslich. Ähnlich wie Mannitol hat Erythritol die vorerwähnten positiven Eigenschaften und hat gegenüber anderen Alditolen wie Sorbit, Maltit, Lactit und Isomalt den Vorteil einer besonders hohen digestiven Toleranz. Bei Abtragsmessungen hat sich überraschenderweise gezeigt, dass trotz der etwas geringeren Dichte gegenüber Mannitol die Abrasivität von Erythritol bei gleicher Teilchengröße leicht erhöht war. Dies liegt vermutlich in der unterschiedlichen Kristallmorphologie von Erythritol, die zur Ausbildung einer scharfkantigeren Oberfläche der Teilchen führt. Besonders vorteilhaft ist Erythritol, da es in den Mischkammern von handelsüblichen Pulverstrahlgeräten zuverlässig bis zu unteren Teilchengrößen von etwa 12 µm verwirbelt werden kann. Obwohl die Wirksamkeit bei der Reinigung von Zahnoberflächen ähnlich gut ist wie bei größeren Mannitol- oder Glycin-Pulvern, ist die Abrasivität deutlich geringer als bei einem Pulver aus Glycin mit einer mittleren Korngröße von 20 µm, oder Mannitol mit einer mittleren Korngröße von 23 µm. Somit ist Erythritol besonders schonend zu Dentin.

Weiterhin eignet sich ein Pulver aus Xylitol, ein farbloses, süß schmeckendes Kristall mit einer molaren Masse von 152,15 g/mol und einer geringen Dichte von 0,77 g/cm³. Es ist in Wasser leicht löslich und hat den Vorteil, dass es eine antikariogene Wirkung entfaltet. Es ist ein natürlicher Zuckeralkohol, der in vielen Gemüsesorten und Früchten (u. a. in Pflaumen, Erdbeeren oder Himbeeren) auftritt. Während des Kohlenhydratabbaus wird Xylitol im Körper des Menschen in der Leber täglich hergestellt und hat aufgrund seiner süßlichen Eigenschaft durchwegs positive Eigenschaften eines Nahrungsergänzungsmittels. Xylitol ist ebenso für Diabetiker geeignet.

Ein weiteres geeignetes Alditol ist Sorbit mit einer Dichte von 1,49 g/cm³ und einer molaren Masse von etwa 182,2 g/mol. Sorbit bildet farblose, geruchlose und hygroskopische Nadeln mit süßem Geschmack und lässt sich aus Mais- oder Weizenstärke herstellen. Es ist sehr leicht löslich in Wasser und eignet sich daher besonders gut für das Pulver aufgrund seiner vorerwähnten Eigenschaften.

Die Erfindung betrifft auch eine Verwendung eines Pulvergemischs zur Herstellung eines Mittels zur Pulverstrahl reinigung von Zahnoberflächen, bevorzugt Dentin.

Das Pulvergemisch weist mindestens zwei der vorgenannten Pulver auf, wobei jedes der Pulver in einem Massenanteil von nicht weniger als 2 %, bevorzugt nicht weniger als 10 %, besonders bevorzugt nicht weniger als 20 %, bezogen auf die Gesamtmasse der vorerwähnten Pulver, vorliegt. Diese Massenangaben beziehen sich somit ausschließlich auf die abrasiv wirkenden Pulver der vorgenannten Erfindung, wobei der Pulvermischung natürlich noch weitere Bestandteile zugefügt werden können. Ein geeignetes Mittel zur Pulverstrahlreinigung von Zahnoberflächen besteht somit aus dem erfindungsgemäßen Pulver oder dem erfindungsgemäßen Pulvergemisch zuzüglich ggf. weiterer Bestandteile, wie beispielsweise feinteilige Stoffe wie Kieselgel, Bleichmittel, Analgetika, Bakteriozide oder Geschmacksstoffe, die dem Mittel hinzugefügt werden. Weiterhin kann dem Mittel Luft und Wasser hinzugefügt werden, um es mittels eines Pulverstrahlgeräts auf die zu bearbeitenden Zahnflächen aufbringen zu können.

Das Pulvergemisch hat darüber hinaus mindestens zwei der vorerwähnten abrasiven Pulver mit einem Teilchenanteil (d. h. Stoffmenge) von nicht weniger als 5 %, bevorzugt nicht weniger als 10 %, besonders bevorzugt nicht weniger als 20 %, bezogen auf die Gesamtteilchenanzahl (d. h. Gesamtstoffmenge) der Pulver nach den vorerwähnten bevorzugten Ausführungsformen. Ein besonders bevorzugtes Pulvergemisch weist Mannitol mit einem Massenanteil von etwa 30 % und Erythritol mit einem Massenanteil von etwa 70 % auf. Dies entspricht einem Stoffmengenverhältnis (d. h., Teilchenverhältnis) von etwa 1:6.

Bei der vorliegenden Erfindung hat sich überraschenderweise gezeigt, dass es möglich ist, einem sehr feinen und leichten Pulver, wie zum Beispiel Erythritol, mit einer mittleren Korngröße von 12 µm, Anteile eines weiteren Pulvers mit etwas größeren Teilchen zuzumischen, wie beispielsweise Mannitol mit einer mittleren Korngröße von ca. 20 µm. Durch die Einlagerung der größeren Teilchen kann der Luftstrom in der Pulverkammer leichter das Pulver aufmischen und die Gefahr einer Kompaktierung, d. h., einer Bildung von verdichteten Bereichen innerhalb der Pulverkammer wird minimiert, d. h., die Verwirbelungsfähigkeit des eingesetzten Pulvergemischs ist nach wie vor gut, während gleichzeitig ein besonders feinteiliges Pulver auf der Zahnoberfläche auftritt, wodurch insbesondere die empfindlichen Zahnwurzeloberflächen geschont werden. Die Abrasivität des Pulvergemischs vergrößert sich dadurch nur unwesentlich, da der Anteil der zugemischten größeren Teilchen nicht all zu hoch ist und das Pulver mit den größeren Partikeln auch aufgrund der Morphologie der Teilchenoberfläche weniger abrasiv wirkt.

Während beim Stand der Technik einem abrasiv wirkenden (Primär-)Pulver weitere feinteilige Stoffe, wie beispielsweise Kieselgel, zur Verbesserung der Rieselfähigkeit zugesetzt wurden, hat die vorliegende Erfindung erkannt, dass die Anforderungen an die Verwirbelungsfähigkeit im Pulverstrahlgerät und die Abrasivität an der Zahnoberfläche durch ein Pulvergemisch erzielt werden kann, das beide konkurrierende Anforderungen, also Verwirbelungsfähigkeit und Abrasivität, gleichermaßen erfüllt.

### Beispiele:

Das Pulver bzw. das Pulvergemisch wurde mit herkömmlichem Pulver aus sehr feinem Natriumbicarbonat verglichen. Derzeit ist Natriumbicarbonat mit einer mittleren Korngröße von etwa 65 µm das am weitesten verbreitete Pulver zur Reinigung von Zahnoberflächen, insbesondere Zahnschmelz. Aufgrund seiner deutlich höheren Abrasivität bei empfindlichen Zahnoberflächen, wie zum Beispiel am Zahnhals, wird dieses Pulver in der Regel dort nicht eingesetzt. Allerdings ist ein solches Natriumbicarbonat-Pulver bis zu einer Pulvergröße mit einer mittleren Korngröße von 10 µm in einem handelsüblichen Pulverstrahlgerät noch zu verarbeiten. Jedoch ist die Abrasivität auch bei solchen kleinen Teilchen immer noch erkennbar höher als bei dem erfindungsgemäßen Erythritol-Pulver mit einer mittleren Korngröße von ca. 12 µm.

Die Versuche wurden daher mit einem handelsüblichen Pulverstrahlgerät (AirFlow S1, EMS Electro Medical Systems SA, Schweiz) durchgeführt. Als Ersatz für den empfindlichen Teil einer Zahnoberfläche kam bei den vorliegenden Versuchen der Kunststoff PEEK GF 30 zum Einsatz, d. h., ein Polyetheretherketon mit einem 30% -igen Glasfaseranteil. Die Düse des Pulverstrahlgeräts wurde in einem Abstand von ca. 2 mm über der Kunststoffoberfläche angeordnet und der Pulverstrahl wurde jeweils 10 Sekunden lang aktiviert. Die Tiefe des mit dem jeweiligen Pulver ausgearbeiteten Bereichs der Kunststoffoberfläche wurde vermessen und dabei als Maß für das Abtragsverhalten bzw. die Abrasivität gewertet. Zum Vergleich wurde auch ein Pulver aus Glycin unterschiedlicher mittlerer Korngrößen herangezogen.

**Tabelle 1: Abrasivität verschiedener Pulver**

| Mittlere Korngröße | Ø 65 µm | Ø 20 µm | Ø 12 µm |
|---|---|---|---|
| Natriumbicarbonat | 10 | 6 | 3 |
| Glycin | 6 | 3 | - |
| Mannitol | 12 | 4 (*) | - |
| Erythritol | 14 | 6 | 2 |
| Mannitol (30%) /Erythritol (70%) | | 3 | |

| | | | |
|---|---|---|---|
| (*)bei Teilchengrößen von 23 µm Ein Wert von 10 entspricht den Eigenschaften von Natriumbicarbonat bei Ø 65 µm "-" bedeutet, dass das Pulver mit dem herkömmlichen Gerät nicht zu fördern war | | | |

**Tabelle 2: Mischfähigkeit verschiedener Pulver**

| Mittlere Korngröße | Ø 65 µm | Ø 20 µm | Ø 12 µm |
|---|---|---|---|
| Natriumbicarbonat | 10 | 10 | 5 |
| Glycin | 10 | 9 | - |
| Mannitol | 10 | 10 ^{(*)} | - |
| Erythritol | 10 | 10 | 8 |
| Mannitol (30%) /Erythritol (70%) | | 9 | |

| | | | |
|---|---|---|---|
| ^{(*)} bei Teilchengrößen von ca. 23 µm Ein Wert von 10 entspricht den Eigenschaften von Natriumbicarbonat bei 0 65 µm "-" bedeutet, dass das Pulver mit dem herkömmlichen Gerät nicht zu fördern war | | | |

Die beigefügten Tabellen 1 und 2 zeigen die Abrasivität bzw. die Mischfähigkeit der untersuchten Pulver gegenüber den herkömmlichen Pulvern aus Natriumbicarbonat bzw. Glycin mit einer mittleren Korngröße von etwa 65 µm. Der Wert 10 entspricht dabei den entsprechenden Eigenschaften von Natriumbicarbonat. Der Tabelle 1 ist zu entnehmen, dass die Abrasivität von Pulver aus Mannitol mit einer mittleren Korngröße von 23 µm geringer ist als die Abrasivität von Natriumbicarbonat, obgleich sich die Mischfähigkeit nach Tabelle 2 nicht verschlechtert. Noch kleinere Teilchengrößen von beispielsweise 12 µm lassen sich bei der Verwendung von Erythritol in handelsüblichen Pulverstrahlgeräten verarbeiten, wobei die Abrasivität geringer und die Mischfähigkeit besser ist als von herkömmlichem Natriumbicarbonat.

Besonders erstaunlich ist die Abrasivität bzw Mischfähigkeit eines Pulvergemischs aus einem Massenanteil von 30 % Mannitol mit mittleren Korngrößen von ca. 23 µm und einem Massenanteil von 70 % Erythritol mit mittleren Korngrößen von ca 12 µm (d. h. Stoffmengenverhältnis von ca. 1:6, d.h. das Verhältnis der Anzahl der Teilchen nimmt dabei einen Wert von etwa 1:6 (Mannitol:Erythritol) an), die in etwa der Abrasivität bzw. Mischfähigkeit von Glycin entspricht, obgleich das Pulvergemisch erhebliche Vorteile gegenüber Glycin aufweist. So hat dieses Pulvergemisch eine geringere Korrosionswirkung auf Stahl und bewirkt einen geringeren Verschleiß der Strahldüse als Glycin. Des Weiteren ist das Pulvergemisch nicht zu toxisch und kann nicht - wie bei Glycin - beim Kontakt mit der Haut zu Irritationen führen. Die Mischung ist direkt herstellbar und muss nicht - wie Glycin - gemahlen und gesiebt werden, wodurch sich die Herstellungskosten erheblich reduzieren lassen. Die Pulvermischung ist nicht kariogen und kann bei Diabetikern eingesetzt werden.

Besonders vorteilhaft ist die erfindungsgemäße Verwendung eines Pulvers, da dessen Reinigungswirkung erheblich besser ist als bei herkömmlichen Pulvern, d. h., die Abtragung von Zahnbelägen erfolgt schneller, vermutlich aufgrund der Kristallmorphologie von Erythritol. Hierzu wurden die folgenden Versuche angestrengt:

Für die Bestimmung des Wertes der Reinigungswirkung der vorbezeichneten Vergleichspulver und der erfindungsgemäßen Pulver wurde von einer beschichteten Platte jeweils ein 1 cm x 0,5 cm großes Areal solange mit dem aus einem Pulverstrahlgerät kommenden Pulver bearbeitet, bis diese Fläche vollständig von ihrer Beschichtung befreit war. Die dazu benötigte Zeit wurde gemessen. Jeder Versuch wurde zehnmal wiederholt, um statische Schwankungen zu berücksichtigen. Die Versuche wurden wieder mit einem handelsüblichen Pulverstrahlgerät (AirFlow S1, EMS Electro Medical Systems SA, Schweiz) durchgeführt.

Der Reinigungswirkung des Natriumbicarboncat-Pulvers mit 65 µm Teilchendurchmesser ist zur besseren Vergleichbarkeit, wie in den anderen Versuchen zuvor, der Wert 10 zugeordnet. Die ermittelten Zeiten für die anderen Pulver sind mit diesem Wert normiert worden und die Kehrwerte in Tabelle 3 eingetragen. Ein hoher Punktewert steht demnach für eine gute Reinigungswirkung.

**Tabelle 3: Reinigungswirkung verschiedener Pulver**

| | Ø 65 µm | Ø 20 µm | Ø 12 µm |
|---|---|---|---|
| Natriumbicarbonat | 10 | 11 | 10 |
| Glycin | 8 | 8 | - |
| Mannitol | 13 | 6 | - |
| Erythritol | 21 | 19 | 12 |
| Mannitol (30%) /Erythritol (70%) | - | 10 | |

| | | | |
|---|---|---|---|
| Ein Wert von 10 entspricht den Eigenschaften von Natriumbicarbonat bei Ø 65 µm "-" bedeutet, dass das Pulver mit dem herkömmlichen Gerät nicht zu fördern war | | | |

Die Reinigungswirkung der untersuchten Pulver ist überraschenderweise für fast alle Pulversorten im Vergleich zum Standardpulver (Natriumbicarbonat mit mittleren Korngrößen von ca. 65 µm) entweder besser oder zumindest ähnlich. Lediglich das Mannitol mit einer Korngröße von 20 µm fällt etwas ab. Auch die untersuchten Glycin-Pulver erreichen nicht die Leistung des Standardpulvers. Die Pulver aus Erythritol hingegen zeigen eine hervorragende Reinigungswirkung, die immer über dem des Standardpulvers lag. Auch die Reinigungswirkung der Mischung aus Mannitol und Erythritol ist der des Standardpulvers äquivalent.

Unter Berücksichtigung der ermittelten Werte für die Abrasivität, die Mischfähigkeit und die Reinigungswirkung sind die Pulversorten Erythritol 12 µm und die Mischung aus Erythritol und Mannitol den bekannten Pulvern für empfindliche Zahnoberflächen überlegen. Wird ein Abrasivitätswert von 3 als obere Grenze für empfindliche Zahnoberflächen festgelegt, so erfüllen dies die genannten Pulver bzw. liegen noch darunter. Gleichzeitig zeigen sie eine bessere Reinigungswirkung als bekannte Pulver aus Glycin.

## Patentansprüche

1. Verwendung eines Pulvers, welches ein Alditol aufweist, zur Herstellung eines Mittels zur Pulverstrahlreinigung von Zahnoberflächen, bevorzugt Dentin.

2. Verwendung nach Anspruch 1, wobei die mittlere Korngröße des Pulvers nicht größer ist als 45 µm, bevorzugt nicht größer ist als 35 µm, besonders bevorzugt zwischen 10 µm und 30 µm liegt.

3. Verwendung nach Anspruch 1 oder 2, **dadurch gekennzeichnet, dass** das Pulver eine Dichte von nicht mehr als 2,0 g/cm³, bevorzugt nicht mehr als 1,8 g/cm³, besonders bevorzugt nicht mehr als 1,55 g/cm³ hat.

4. Verwendung nach einem der vorhergehenden Ansprüche, **dadurch gekennzeichnet, dass** das Pulver eine Mohssche Härte von nicht mehr als 4, bevorzugt von nicht mehr als 3,5, besonders bevorzugt von nicht mehr als 2,5 hat.

5. Verwendung nach einem der vorhergehenden Ansprüche, **dadurch gekennzeichnet, dass** das Pulver eine molare Masse von nicht weniger als 90 g/mol, bevorzugt nicht weniger als 110 g/mol, besonders bevorzugt nicht weniger als 120 g/mol aufweist.

6. Verwendung nach einem der vorhergehenden Ansprüche, **dadurch gekennzeichnet, dass** das Pulver ein Mannitol oder Erythritol aufweist.

7. Verwendung eines Pulvergemisches zur Herstellung eines Mittels zur Pulverstrahlreinigung von Zahnoberflächen, bevorzugt Dentin, wobei das Pulvergemisch mindestens zwei Pulver aufweist, welche jeweils ein Alditol enthalten, und wobei jedes der Pulver in einem Massenanteil von nicht weniger als 2%, bevorzugt nicht weniger als 10%, besonders bevorzugt nicht weniger als 20%, bezogen auf die Gesamtmasse der Pulver, vorliegt.

8. Verwendung nach Anspruch 7, **dadurch gekennzeichnet, dass** jedes der Pulver mit einem Teilchenanteil von nicht weniger als 5%, bevorzugt nicht weniger als 10%, besonders bevorzugt nicht weniger als 20%, bezogen auf die Gesamtteilchenanzahl der Pulver welche Alditol enthalten, vorliegt.

9. Verwendung nach Anspruch 7 oder 8, wobei die mittlere Korngröße der Pulver nicht größer ist als 45 µm, bevorzugt nicht größer ist als 35 µm, besonders bevorzugt zwischen 10 µm und 30 µm liegt.

10. Verwendung nach einem der Ansprüche 7 - 9, **dadurch gekennzeichnet, dass** die Pulver eine Dichte von nicht mehr als 2,0 g/cm³, bevorzugt nicht mehr als 1,8 g/cm³, besonders bevorzugt nicht mehr als 1,55 g/cm³ haben.

11. Verwendung nach einem der Ansprüche 7 -10, **dadurch gekennzeichnet, dass** die Pulver eine Mohssche Härte von nicht mehr als 4, bevorzugt von nicht mehr als 3,5, besonders bevorzugt von nicht mehr als 2,5 haben.

12. Verwendung nach einem der Ansprüche 7-11, **dadurch gekennzeichnet, dass** die Pulver eine molare Masse von nicht weniger als 90 g/mol, bevorzugt nicht weniger als 110 g/mol, besonders bevorzugt nicht weniger als 120 g/mol aufweisen.

13. Verwendung nach einem der Ansprüche 7 -12, **dadurch gekennzeichnet, dass** das Pulvergemisch ein Mannitol oder Erythritol aufweist und bevorzugt Mannitol mit einem Massenanteil von 30% und Erythritol mit einem Massenanteil von 70% aufweist.

14. Verwendung nach einem der Ansprüche 1 - 13" wobei das Mittel zur Pulverstrahlreinigung von Zahnoberflächen weitere feinteilige Stoffen wie Kieselgel, Bleichmittel, Analgetika, Bakteriozide und/oder Geschmacksstoffe enthält.

15. Verwendung nach einem der Ansprüche 1 -14, wobei das Mittel zur Pulverstrahlreinigung von Zahnoberflächen zusätzlich Luft und Wasser aufweist und mittels eines Pulverstrahlgeräts auf die zu bearbeitenden Zahnflächen aufgebracht wird.

## Claims

1. Use of a powder which contains an alditol for the production of a medium for pulverulent jet cleaning or powder blasting of dental surfaces, preferably dentin.

2. Use according to claim 1, wherein the mean grain size of the powder is no larger than 45 µm, preferably no larger than 35 µm, much preferred between 10 µm and 30 µm.

3. Use according to claim 1 or 2, **characterized in that** the powder has a density of no more than 2.0 g/cm³, preferably no more than 1.8 g/cm³, much preferred no more than 1.55 g/cm³.

4. Use according to any one of the foregoing claims, **characterized in that** the powder has Mohs hardness of no more than 4, preferably no more than 3.5, much preferred no more than 2.5.

5. Use according to any one of the foregoing claims, **characterized in that** the powder has a molar mass of no less than 90 g/mol, preferably no less than 110 g/mol, much preferred no less than 120 g/mol.

6. Use according to any one of the foregoing claims, **characterized in that** the powder contains a mannitol or erythritol.

7. Use of a powder mixture for the production of a medium for pulverulent jet cleaning or powder blasting of dental surfaces, preferably dentin, wherein the power mixture comprises at least two powders which each contain an alditol, and wherein each of the powders is present in a mass fraction of no less than 2%, preferably no less than 10%, much preferred no less than 20%, based on the overall mass of the powders.

8. Use according to claim 7, **characterized in that** each of the powders is present with a particle fraction of no less than 5%, preferably no less than 10%, much preferred no less than 20%, based on the total number of particles of the powders which contain alditol.

9. Use according to any one of claims 7 or 8, wherein the mean grain size of the powders is no larger than 45 µm, preferably no larger than 35 µm, much preferred between 10 µm and 30 µm.

10. Use according to any one of claims 7-9, **characterized in that** the powders have a density of no more than 2.0 g/cm³, preferably no more than 1.8 g/cm³, much preferred no more than 1.55 g/cm³.

11. Use according to any one of claims 7-10, **characterized in that** the powders have Mohs hardness of no more than 4, preferably no more than 3.5, much preferred no more than 2.5.

12. Use according to any one of claims 7-11, **characterized in that** the powders have a molar mass of no less than 90 g/mol, preferably no less than 110 g/mol, much preferred no less than 120 g/mol.

13. Use according to any one of claims 7-12, **characterized in that** the powder mixture comprises a mannitol or erythritol, and preferably mannitol with a mass fraction of 30% and erythritol with a mass fraction of 70%.

14. Use of a powder according to any one of claims 1-13, wherein the medium for pulverulent jet cleaning or powder blasting of dental surfaces contains further finely particled agents, like silica gel, bleaching agents, analgetics, bacteriocides and/or flavor additives.

15. Use according to any one of claims 1-14, wherein the medium for pulverulent jet cleaning or powder blasting of dental surfaces additionally comprises air and water and is applied onto the dental surface to be treated by means of a powder-jet device.

## Revendications

1. Utilisation d'une poudre, qui comprend un alditol, pour la fabrication d'un agent pour le nettoyage au jet de poudre de surfaces dentaires, de préférence de dentine.

2. Utilisation selon la revendication 1, dans laquelle la granulométrie moyenne de la poudre n'est pas supérieure à 45 µm, de préférence pas supérieure à 35 µm, de manière préférée entre 10 µm et 30 µm.

3. Utilisation selon la revendication 1 2, **caractérisée en ce que** la poudre a une densité qui n'est pas supérieure à 2,0 g/cm³, de préférence pas supérieure à 1,8 g/cm³, de manière particulièrement préférée pas supérieure à 1,55 g/cm³.

4. Utilisation selon l'une des revendications précédentes, **caractérisée en ce que** la poudre a une dureté de Mohs qui n'est pas supérieure à 4, de préférence pas supérieure à 3,5, et de manière particulièrement préférée pas supérieure à 2,5.

5. Utilisation selon l'une des revendications précédentes, **caractérisée en ce que** la poudre a une masse molaire qui n'est pas inférieure à 90 g/mole, de préférence pas inférieure à 110 g/mole, de manière particulièrement préférée pas inférieure à 120 g/mole.

6. Utilisation selon l'une des revendications précédentes, **caractérisée en ce que** la poudre comprend un mannitol ou un érythritol.

7. Utilisation d'un mélange de poudre pour la fabrication d'un agent pour le nettoyage au jet de poudre de surfaces dentaires, de préférence de dentine, dans laquelle le mélange de poudre comprend au moins deux poudres, qui contiennent chacune un alditol, et dans laquelle chacune des poudres se présente dans une proportion en masse qui n'est pas inférieure à 2 %, de préférence pas inférieure à 10 %, de manière particulièrement préférée pas inférieure à 20 %, par référence à la masse totale de la poudre.

8. Utilisation selon la revendication 7, **caractérisée en ce que** chacune des poudres se présente avec une proportion de particules qui n'est pas inférieure à 5 %, de préférence pas inférieure à 10 %, de manière particulièrement préférée pas inférieure à 20 %, par référence au nombre de totales des particules de poudre qui contiennent un alditol.

9. Utilisation selon la revendication 7 ou 8, dans laquelle la granulométrie moyenne des poudres n'est pas supérieure à 45 µm, de préférence pas supérieure à 35 µm, de manière particulièrement préférée entre 10 µm et 30 um.

10. Utilisation selon l'une des revendications 7 à 9, **caractérisée en ce que** les poudres ont une densité qui n'est pas supérieure à 2,0 g/cm3, de préférence pas supérieure à 1,8 g/cm3, de manière particulièrement préférée pas supérieure à 1,55 g/cm3.

11. Utilisation selon l'une des revendications 7 à 10, **caractérisée en ce que** les poudres ont une dureté de Mohs qui n'est pas supérieure à 4, de préférence pas supérieure à 3,5, et de manière particulièrement préférée pas supérieure à 2,5.

12. Utilisation selon l'une des revendications 7 à 11, **caractérisée en ce que** les poudres ont une masse molaire qui n'est pas inférieure à 90 g/mole, de préférence pas inférieure à 110 g/mole, de manière particulièrement préférée pas inférieure à 120 g/mole.

13. Utilisation selon l'une des revendications 7 à 12, **caractérisée en ce que** le mélange de poudre comprend un mannitol ou un érythritol, et de préférence du mannitol avec une proportion en masse de 30 % et de l'érythritol avec une proportion en masse de 70 %.

14. Utilisation selon l'une des revendications 1 à 13, dans laquelle l'agent pour le nettoyage au jet de poudre de surfaces dentaires contient d'autres produits en fines particules tels que gel de silice, agent de blanchiment, analgésiques, bactéricides et/ou aromates.

15. Utilisation selon l'une des revendications 1 à 14, dans laquelle l'agent pour le nettoyage au jet de poudre de surfaces dentaires contient additionnellement de l'air et de l'eau et est appliqué au moyen d'un appareil à jet de poudre sur les surfaces dentaires à traiter.
